# EUROPEAN PATENT SPECIFICATION

(11) **EP 1 294 300 B1**
(45) Date of publication and mention of the grant of the patent: **03.11.2004**
(21) Application number: 01943669.0
(22) Date of filing: 27.06.2001
(51) Int. Cl.: A61B 19/00

(54) **APPARATUS FOR POSITIONING A SURGICAL INSTRUMENT**
GERÄT ZUR POSITIONIERUNG EINES CHIRURGISCHEN INSTRUMENTS
DISPOSITIF DE POSITIONNEMENT D'UN INSTRUMENT CHIRURGICAL

(30) Priority: 28.06.2000 GB 0015683
(43) Date of publication of application: 26.03.2003
(73) Proprietor: Depuy International Limited, Beeston, Leeds LS11 8DT (GB)
(72) Inventor: WAHRBURG, Jürgen, 51709 Mareinheide (DE)
(74) Representative: Belcher, Simon James
(86) International application number: PCT/GB2001/002899
(87) International publication number: WO 2002/000131

(56) References cited:
- US-A- 5 142 930
- US-A- 5 695 500

## Description

This invention relates to apparatus for positioning a surgical instrument during a surgical orthopaedic procedure.

WO-A-98/27887 discloses apparatus for use positioning a surgical instrument using a robot which moves the surgical instrument within the apparatus coordinate system according to program instructions from a control computer. The apparatus includes a sensor in the form of a manually movable sensor arm or of a remote receiver (for example in the form of an optical signal receiver which can receive light signals from light emitting diodes) which can be used to define a coordinate system for the apparatus and to locate the patient's bone on which the procedure is to be performed within that coordinate system. Defining the coordinate system in this way can involve locating markers or anatomic features on the patient's bone. For example, a sensor comprising a manually movable arm can be moved to contact markers on the bone. When the sensor comprises a receiver, it can receive information from bone markers which indicate their position. By establishing the location of the instrument relative to the sensor, the apparatus can work from the information from the locating markers to establish the location of the bone relative to that of the instrument. Instructions to control the surgical instrument can then be provided by the control computer using instructions supplied to it by the physician.

The disclosed apparatus can use information from one or more tracking markers (for example LED markers) on the bone to monitor movement of the bone during the surgical procedure which can then be taken into account to modify the signal which is supplied by the computer to the robot so that the instrument continues to be appropriately positioned relative to the bone.

The signal that is supplied by the computer to the robot in such apparatus relies on the robot being able to deliver the instrument to a desired location. The apparatus therefore relies on precise calibration of the robot in order for the surgical instrument to be positioned accurately. These calibration requirements can require that the robot be recalibrated regularly. This can be time consuming. However, failure to recalibrate when required can lead to inaccuracies in the positioning of the instrument which can jeopardise the success of the surgical procedure.

An apparatus according to the preamble of claim 1 is disclosed in US-A-5 695 500. The present invention provides apparatus for positioning a surgical instrument in which the computer for generating the position signal to the robot uses a process which involves minimising the difference between the true position of the instrument and the said desired location.

Accordingly, in one aspect, the invention provides apparatus for positioning a surgical instrument during a surgical orthopaedic procedure relative to the coordinate system of the apparatus, which comprises:
a. a surgical instrument,
b. a robotic control system for moving the surgical instrument within the apparatus coordinate system according to program instructions,
c. an instrument sensor which is fixed relative to the instrument to indicate the true position of the instrument within the apparatus coordinate system,
d. a reference sensor which can be fixed to a patient's bone to indicate the position of the patient's bone within the apparatus coordinate system,
e. a detector for monitoring the positions of the instrument sensor and the reference sensor, and
f. a signal processor which receives position signals from the detector, and which generates a desired position signal to the robotic control system to position the surgical instrument at a desired location relative to the reference sensor, by a process which involves minimising the difference between the true position of the instrument and the said desired location.

The apparatus of the present invention has the advantage that the requirement for accurate calibration of a robotic control system is reduced because the apparatus controls the position of the surgical instrument directly relative to the position of the patient's bone. The apparatus of the invention therefore allows errors in the calibration of the robotic control system which otherwise might lead to the surgical instrument being positioned wrongly relative to the bone to be taken into account, and preferably also corrected.

A further significant advantage of the apparatus of the invention is that the signal that is generated to the robotic control system position can take into account movement of the patient's bone during the procedure so that the surgical instrument can continue to be positioned accurately relative to the bone.

Preferably, the process by which the signal processor generates the desired position signal to the robotic control system is an iterative process so that the difference between the true position of the instrument and the said desired location can be calculated more than once with adjustments to the desired position signal as necessary after each calculation.

Preferably, the instrument sensor or the reference sensor or each of them generates a signal to indicate the position of the instrument or the bone (as the case might be) which can be detected by the detector. For example, the signal might be an optical signal such as might be generated by one or more light emitting diodes. Other forms of signal can be used, for example as generated using inductive or ultrasonic processes. The signal can be appropriately coded so that the detector can identify the signal and so identify the sensor from which it is transmitted. Preferably, a sensor generates more then one signal, more preferably at least two signals, especially at least three signals, the signal generators (for example, light emitting diodes) being arranged in a fixed spatial arrangement. The provision of several signal generators has the advantage that the apparatus is able to determine both the location and orientation of the sensor. Locating systems with such sensors are known. For example, a sensor might include a small plate with four or six infrared light emitting diodes as signal generators arranged at its comers. The light impulses emitted from the diodes are received be the detector which has two, three or more separate light sensitive receiving elements. The position and orientation of the sensor can be calculated by mathematical evaluation of the received signals, making use of the known geometrical arrangements of the receiving elements and the diodes on the plate.

It is important for movement of the reference sensor relative to the patient's anatomy to be minimised. Generally, the reference sensor will be fixed to a bone, especially the bone on which the surgical instrument of the apparatus is to operate. The reference sensor can have a threaded shank, and can then be fixed to the bone by a technique which involves a first step of drilling a hole in the bone, and then screwing the shank of the sensor into the drilled hole.

The robotic control system can provide an arm which includes a mounting plate on which the surgical instrument is mounted. The arm is jointed so that the instrument can be moved. Other control systems can be used which provide for movement of the instrument in different ways. For example, the instrument might be mounted on a carriage which can slide along a primary shaft. Additional degrees of freedom can be provided by secondary shafts on which the primary shaft can slide. Preferably, the robotic control system provides at least three degrees of freedom of movement of the instrument. Preferred systems can provide at least six degrees of freedom of movement.

The robotic control system can include a stationary base part to which an arm for the surgical instrument is fixed, so that the arm and an instrument mounted on it can be moved under program instructions. The robotic control system will also generally include a controller with appropriate processor components for generating instructions for causing the instrument to move. The controller can be combined with the base part of the control system on which the arm is fixed. Frequently however the controller will be provided separate from the base part of the control system.

The apparatus can include markers for fixing to a patient prior to a scanning operation for generating an image of the bone, and which can be referred to in the surgical procedure to relate the position of the patient's bone within the apparatus coordinate system as indicated by the reference sensor to a previously generated image of the bone. Such markers can serve as reference points for use in connection with X-ray or computer tomograph scans. The use of such markers (often referred to as fiducial markers) is established. Conventionally, they are fixed to a bone by means of screw threads or similar features. They can be used with a probe which is connected to a navigation computer which can contact each marker in turn to identify it for the computer which can then relate its location relative to the previously generated bone image. The position of the probe can be monitored using mechanically, for example by mounting it on a jointed arm in which movement of the joints can be measured. The probe is then moved manually from an initially determined home position to each of the markers and the position of each marker is determined relative to the home position by measurement of the movement of the probe. Such measurement systems are known generally.

Other techniques for determining the bone location can be used. For example, the bone location can be determined using natural anatomical features instead of or in conjunction with fixable markers.

Preferably, the apparatus of the invention includes a registration probe for determining the location of the patient's bone within the apparatus coordinate system by contacting the bone at predetermined points thereon. The points can be defined by implanted markers or by anatomical landmark markers, for example provided by specific distinctive locations on the bone surface or by distinctive bone contours.

In a preferred arrangement, the position of the probe is monitored using the detector, for example by including on the probe at least one signal generator (and preferably more as discussed above in relation to the instrument and reference sensors) on it.

It is important for the position of the bone to remain unchanged while its location within the apparatus coordinate system is initially determined. However, once the location of the bone has been determined, the apparatus of the present invention presents the advantage that movement of the bone can be accommodated by consequent movement of the surgical instrument.

Generally, the detector is fixed within the apparatus coordinate system and the apparatus coordinate system is defined relative to the position of the detector. Preferably, the detector is fixed relative to the robotic control system for moving the surgical instrument. However, in order to avoid the possibility of inaccuracies being introduced due to small amounts of movement of the detector relative to the robot control system, it can be preferred for the apparatus to include a robotic control system sensor to indicate the true position of the robotic control system within the apparatus coordinate system. The robotic control system sensor can have features of the instrument and reference sensors discussed above.

Embodiments of the present invention will now be described by way of example with reference to the accompanying drawings, in which:
Figure 1 is a schematic representation of the apparatus of the present invention.
Figure 2 is a front view of a sensor which could be an instrument sensor or a reference sensor.

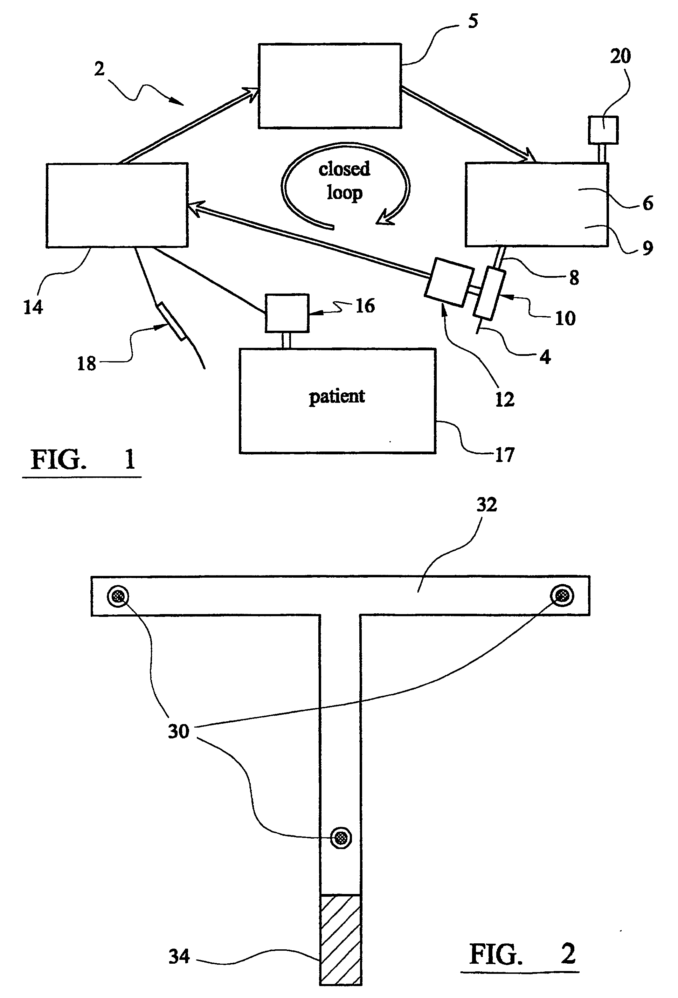

Referring to the drawings, Figure 1 is a schematic representation of apparatus 2 which can be used to position a surgical instrument 4. The apparatus includes a computer 5 and a robotic control system 6 which can receive instructions generated by the computer to cause the instrument to move. The control system includes a robot arm (or other robotic system) 8 which has a mounting plate 10 at its end on which the instrument 4 is mounted. The robot arm 8 is fixed to a stationary base part 9 of the control system. Instructions to move the instrument are interpreted by the robotic control system and cause the robot arm, with the instrument mounted on it, to move relative to the stationary base part 9. Preferably, the robot arm has at least three joints, more preferably six joints, to enable sufficient freedom of movement of the mounting plate. Robotic control systems of this general kind are known, for example as referred to in US-6033415 and WO-98/27887.

The robot arm has an instrument sensor 12 on the mounting plate and is therefore fixed relative to the instrument. The instrument sensor comprises a plate which has a plurality of light emitting diodes (for example 4 or 6) arranged on it. The sensor generates an instrument signal to indicate the position of the instrument 4.

The apparatus includes an optical detector 14 which can receive the signal generated by the instrument sensor 12 in order to monitor the position of the sensor. The optical detector has at least two (for example three) separate receiving elements. The position and orientation of the instrument sensor 12 (and therefore also of the instrument 4 which is fixed relative to the sensor) can be evaluated based on the known geometrical arrangement of the LEDs on the instrument sensor and of the receiving elements of the detector.

The apparatus also includes a reference sensor 16 which can be fixed to the bone in the patient 17 on which the surgical procedure is to be performed. The reference sensor 16, like the instrument sensor 12, comprises a plate which has a plurality of light emitting diodes arranged on it. Once fixed to the bone, the position and orientation of the reference sensor 16 (and therefore also of the patient's bone which is fixed relative to the sensor) can be evaluated based on the known geometrical arrangement of the LEDs on the sensor and of the receiving elements of the detector.

The apparatus includes a registration probe 18 which can be used to register the position of the patient's bone in the apparatus coordinate system which is defined with respect to the detector 14. Registration of the bone's position is carried out by touching the probe on fiducial markers which had been implanted in the bone prior to a scanning operation (especially an X-ray or CT scan) for generating an image of the bone. The markers can be referred to in the surgical procedure to relate the position of the patient's bone within the apparatus coordinate system as indicated by the reference sensor to the previously generated image of the bone. The use of such markers (often referred to as fiducial markers) with a registration probe is established, when they are used with a probe which is connected to a navigation computer which can be used to contact each marker in turn to identify it for the computer which can then relate its location relative to the previously generated bone image. The position of the probe can be monitored using mechanically, for example by mounting it on a jointed arm in which movement of the joints can be measured. The probe is then moved manually from an initially determined home position to each of the markers and the position of each marker is determined relative to the home position by measurement of the movement of the probe. Such measurement systems are known generally.

Optionally, the apparatus can include a robotic control system sensor 20 to indicate the true position of the robotic control system within the apparatus coordinate system. The robotic control system sensor is mounted on the stationary base part 9 of the robotic control system. The robotic control system sensor, like the instrument sensor 12, comprises a plate which has a plurality of light emitting diodes arranged on it. The position and orientation of the robotic control system sensor 16 (and therefore also of the patient's bone which is fixed relative to the sensor) can be evaluated based on the known geometrical arrangement of the LEDs on the sensor and of the receiving elements of the detector. This enables changes in the position of the robotic control system to be taken into account in the instructions generated by the computer to the robotic control system to ensure that the surgical instrument is positioned appropriately.

Use of the apparatus of the invention can be in accordance with the following sequence of steps:
1. Surgical implantation of fiducial markers in the bone on which the surgical instrument 4 is to operate.
2. Generation of an image of the bone, for example using X-ray or computer tomography scanning equipment (not shown).
3. Planning the surgical procedure which is to be performed, including the steps to be performed using the surgical instrument.
4. Placing the patient on an operating table in such a way that movement of the bone on which the instrument 4 is to operate is minimised.
5. Fixing the reference sensor 16 relative to the bone.
6. Locating the bone in the apparatus coordinate system which is defined with respect to the detector 14 using the probe 18, by contacting the markers on the bone.
7. Performing a matching procedure to align (a) the coordinates of the markers in the image of the bone generated in step 2, with (b) the coordinates of the markers as located using the probe 18 in step 6.
8. Detecting signals from the instrument sensor 12 and the reference sensor 16 by means of the detector 14 and evaluating the position and orientation of the instrument sensor (and therefore also of the instrument 4 which is fixed relative to the sensor), and of the reference sensor (and therefore also of the bone to which the reference sensor is fixed).
9. Generating a signal by means of the computer 5 which is transmitted to the robotic control system 6 to cause the robot arm 8 to move the instrument 5 to a desired position relative to the patient's bone in which the instrument can operate on the bone.
10. Comparing the actual position of the surgical instrument, as determined from the signal received by the detector from the instrument sensor, with the desired position relative the bone.
11. Generating a signal by means of the computer to cause the robot arm to move the instrument so that the difference between the desired position relative to the patient's bone and the instrument's actual position is reduced.
12. Repeating steps 9 and 10 as necessary.

Step 1 of the above procedure can be omitted if anatomical landmark markers (for example provided by specific distinctive locations on the bone surface or by distinctive bone contours) are used instead of implanted markers. Such landmark markers are identified in an image which is generated from a pre-operative examination of the patient (such as that in step 2). They must also be identified during the procedure using a probe (such as the probe 18) to contact the landmark markers, or to generate a plurality of points by following an anatomical contour or surface (in a step analogous to step 6). The use of anatomical landmark markers has advantages in that the patient need not be subjected to in initial surgical procedure by which markers are implanted.

In the event that the patient moves within the apparatus coordinate system, such movement is detected by the detector from the signal transmitted to it by the reference sensor. Such movement might result in a change in the difference between the desired position of the surgical instrument (which is defined with respect to the bone) and the instrument's actual position. This can give rise to additional signals generated by the computer, to cause the robot arm to move the instrument so the movement of the patient is taken into account.

Accurate positioning of the instrument can require that the location of the fixed part of the robotic control system 6 is known accurately since the position of the surgical instrument 4 on the mounting plate 10 is changed in response to a signal which is received by the robotic control system which causes the robot arm 8 to move relative to the fixed part of the system. In the event that the fixed part of the robotic control system moves within the apparatus coordinate system (defined relative to the detector 14), such movement is detected by the detector from the signal transmitted to it by the reference sensor. Such movement might result in a change in the difference between the desired position of the surgical instrument (which is defined with respect to the bone) and the instrument's actual position. This can give rise to additional signals generated by the computer, to cause the robot arm to move the instrument so the movement of the patient is taken into account.

The apparatus of the invention enables errors in the robotic positioning of a surgical instrument due to calibration errors in the robot to be reduced. It therefore reduces the importance of accurate calibration of the robot.

Examples of instruments which can be fixed to the mounting plate 10 include broaches, reamers, and saws.

Figure 2 is a schematic view of a sensor which could be, for example, an instrument sensor or a reference sensor. The sensor has three light emitting diodes 30 which are spaced apart mounted on a support frame 32. The support from has a mounting segment 34 at which it can be affixed to its respective substrate (instrument, patient etc).

## Claims

1. Apparatus for positioning a surgical instrument during a surgical orthopaedic procedure relative to the coordinate system of the apparatus, which comprises:
a. a surgical instrument (4),
b. a robotic control system (6) or moving the surgical instrument (4) within the apparatus coordinate system according to program instructions,
c. an instrument sensor (12) which is fixed relative to the instrument to indicate the true position of the instrument within the apparatus coordinate system,
d. a reference sensor (16) which can be fixed to a patient's bone to indicate the position of the patient's bone within the apparatus coordinate system,
e. a detector (14) for monitoring the positions of the instrument sensor and the reference sensor, and
f. a signal processor which receives position signals from the detector, **characterised in that** the signal processor generates a desired position signal to the robotic control system (6) to position the surgical instrument (4) at a desired location relative to the reference sensor (16), by a process which involves minimising the difference between the true position of the instrument and the said desired location.

2. Apparatus as claimed in claim 1, in which the process by which the signal for the position of the surgical instrument is generated is an iterative process.

3. Apparatus as claimed in claim 1, in which the instrument sensor (12) generates an instrument signal to indicate the position of the surgical instrument (4) which can be detected by the detector.

4. Apparatus as claimed in claim 2, in which the instrument signal is an optical signal.

5. Apparatus as claimed in claim 1, in which the reference sensor (16) generates a signal to indicate the position of the bone which can be detected by the detector.

6. Apparatus as claimed in claim 5, in which the reference signal is an optical signal.

7. Apparatus as claimed in claim 1, which includes markers for fixing to a patient prior to a scanning operation for generating an image of the bone, and which can be referred to in the surgical procedure to relate the position of the patients bone within the apparatus coordinate system as indicated by the reference sensor (16) to a previously generated image of the bone.

8. Apparatus as claimed in claim 1, in which the detector (14) is fixed within the apparatus coordinate system.

9. Apparatus as claimed in claim 1, which includes a robotic control system sensor to indicate the true position of the robotic control system within the apparatus coordinate system.

10. Apparatus as claimed in claim 1, which includes a registration probe for determining the location of the patient's bone within the apparatus coordinate system by contacting the bone at predetermined points thereon.

## Patentansprüche

1. Vorrichtung zum Positionieren eines chirurgischen Instrumentes relativ zu dem Koordinatensystem der Vorrichtung während eines orthopädischen Chirurgieverfahrens, umfassend:
a. ein chirurgisches Instrument (4),
b. ein Robotersteuerungssystem (6) zum Bewegen des chirurgischen Instrumentes (4) entsprechend Programmbefehlen innerhalb des Koordinatensystems der Vorrichtung,
c. einen Instrumentensensor (12), der eine feste Lage relativ zu dem Instrument einnimmt, um die wahre Position des Instrumentes innerhalb des Koordinatensystems der Vorrichtung anzuzeigen,
d. einen Referenzsensor (16), der an einem Knochen des Patienten befestigbar ist, um die Lage des Knochens des Patienten in dem Koordinatensystem der Vorrichtung anzuzeigen,
e. einen Detektor (14) zum Überwachen der Positionen des Instrumentensensors und des Referenzsensors, und
f. einen Signalprozessor, der Positionssignale von dem Detektor empfängt, **dadurch gekennzeichnet, dass** der Signalprozessor ein gewünschtes Positonssignal für das Robotersteuerungssystem (6) erzeugt, um das chirurgische Instrument (4) in eine gewünschte Position relativ zu dem Referenzsensor (16) durch ein Verfahren zu bewegen, das ein Minimieren der Differenz zwischen der wahren Position des Instrumentes und der gewünschten Position beinhaltet.

2. Vorrichtung nach Anspruch 1, **dadurch gekennzeichnet, dass** das Verfahren, durch das das Signal für die Position des chirugischen Instrumentes erzeugt wird, ein iteratives Verfahren ist.

3. Vorrichtung nach Anspruch 1, **dadurch gekennzeichnet, dass** der Instrumentensensor (12) ein Instrumentensignal erzeugt, um die Position des chirugischen Instrumentes (4) anzuzeigen, die durch den Detektor erfassbar ist.

4. Vorrichtung nach Anspruch 2, **dadurch gekennzeichnet, dass** das Instrumentensignal ein optisches Signal ist.

5. Vorrichtung nach Anspruch 1, **dadurch gekennzeichnet, dass** der Referenzsensor (16) ein Signal erzeugt, um die Lage des Knochens anzuzeigen, die durch den Detektor erfassbar ist.

6. Vorrichtung nach Anspruch 5, **dadurch gekennzeichnet, dass** das Referenzsignal ein optisches Signal ist.

7. Vorrichtung nach Anspruch 1, **dadurch gekennzeichnet, dass** sie Marker umfasst, die an einem Patienten vor einem Scanvorgang befestigbar sind, um ein Bild des Knochens zu erzeugen und auf die während des chirugischen Verfahrens Bezug genommen werden kann, um sie mit der Lage des Knochens des Patienten, die durch den Referenzsensor (16) für ein zuvor erzeugtes Bild des Knochens angezeigt wird, innerhalb des Koordinatensystems der Vorrichtung in Bezug zu setzen.

8. Vorrichtung nach Anspruch 1, **dadurch gekennzeichnet, dass** der Detektor (14) innerhalb des Koordinatensystems der Vorrichtung befestigt ist.

9. Vorrichtung nach Anspruch 1, **dadurch gekennzeichnet, dass** sie einen Sensor für das Robotersteuerungssystem umfasst, um die wahre Position des Robotersteuerungssystemes innerhalb des Koordinatensystemes der Vorrichtung anzuzeigen.

10. Vorrichtung nach Anspruch 1, **dadurch gekennzeichnet, dass** sie einen Lagesensor umfasst, um die Lage des Knochens des Patienten innerhalb des Koordinatensystemes der Vorrichtung durch Berühren des Knochens an vorbestimmten Stellen desselben zu bestimmen.

## Revendications

1. Appareil de positionnement d'un instrument chirurgical pendant une procédure orthopédique chirurgicale par rapport au système des coordonnées de l'appareil, qui comprend :
a. un instrument chirurgical (4),
b. un système de commande robotisé (6) destiné à déplacer l'instrument chirurgical (4) à l'intérieur du système des coordonnées de l'appareil en fonction d'instructions de programmation,
c. un capteur d'instrument (12) qui est fixé par rapport à l'instrument pour indiquer la position réelle de l'instrument à l'intérieur du système des coordonnées de l'appareil,
d. un capteur de référence (16) qui peut être fixé sur un os d'un patient pour indiquer la position de l'os du patient à l'intérieur du système des coordonnées de l'appareil,
e. un détecteur (14) destiné à surveiller les positions du capteur d'instrument et du capteur de référence, et
f. un processeur de signal qui reçoit des signaux de positionnement depuis le détecteur, **caractérisé en ce que** le processeur de signal génère un signal de positionnement souhaité au système de commande robotisé (6) afin de positionner l'instrument chirurgical (4) à un emplacement souhaité par rapport au capteur de référence (16), par un processus qui consiste à minimiser la différence entre la position réelle de l'instrument et ledit emplacement souhaité.

2. Appareil selon la revendication 1, dans lequel le processus par lequel le signal pour le positionnement de l'instrument chirurgical est généré dans un processus itératif.

3. Appareil selon la revendication 1, dans lequel le capteur d'instrument (12) génère un signal d'instrument pour indiquer la position de l'instrument chirurgical (4) qui peut être détecté par le détecteur.

4. Appareil selon la revendication 2, dans lequel le signal d'instrument est un signal optique.

5. Appareil selon la revendication 1, dans lequel le capteur de référence (16) génère un signal destiné à indiquer la position de l'os qui peut être détecté par le détecteur.

6. Appareil selon la revendication 5, dans lequel le signal de référence est un signal optique.

7. Appareil selon la revendication 1, qui comprend des marqueurs devant être fixés sur un patient avant l'opération de numérisation destinée à générer une image de l'os, et qui peuvent servir de référence pendant la procédure chirurgicale pour déterminer la position de l'os du patient à l'intérieur du système des coordonnées de l'appareil comme indiqué par le capteur de référence (16) sur une image préalablement générée de l'os.

8. Appareil selon la revendication 1, dans lequel le détecteur (14) est fixé à l'intérieur du système des coordonnées de l'appareil.

9. Appareil selon la revendication 1, qui comprend un capteur de système de commande robotisé destiné à indiquer la position réelle du système de commande robotisé à l'intérieur du système des coordonnées de l'appareil.

10. Appareil selon la revendication 1, qui comprend une sonde d'enregistrement destinée à déterminer l'emplacement de l'os du patient à l'intérieur du système des coordonnées de l'appareil en mettant en contact l'os à des points prédéterminés de celui-ci.
